# EUROPEAN PATENT APPLICATION

(11) **EP 4 656 732 A1**
(43) Date of publication of application: **03.12.2025**
(21) Application number: 23911000.0
(22) Date of filing: 29.12.2023
(51) Int. Cl.: C12P 21/00, C12N 15/63, C07K 14/435

(54) **IN-VITRO CELL-FREE PROTEIN SYNTHESIS SYSTEM FOR INSERTING NON-NATURAL AMINO ACID, AND METHOD AND USE**

(30) Priority: 31.12.2022 CN 202211740484; 02.01.2023 CN 202310000322
(71) Applicant: Kangma-Healthcode (Shanghai) Biotech Co., Ltd, Pudong New Area Shanghai 201321 (CN)
(72) Inventor: GUO, Min, Shanghai 201321 (CN); ZHENG, Li, Shanghai 201321 (CN); XU, Liqiong, Shanghai 201321 (CN); FANG, Pengfei, Shanghai 201321 (CN); YU, Xue, Shanghai 201321 (CN)
(74) Representative: Predazzi, Valentina
(86) International application number: PCT/CN2023/143616
(87) International publication number: WO 2024/141087

(57) **Abstract**

Provided is an in-vitro cell-free protein synthesis system for inserting a non-natural amino acid. The reaction system comprises: (1) a yeast cell extracting solution; (2) a non-natural amino acid; (3) an exogenous orthogonal aminoacyl tRNA synthetase/orthogonal tRNA pair; and (4) a template containing a target protein gene sequence, wherein at least one codon encoding an amino acid in the target protein gene sequence is mutated into a stop codon. By means of the reaction system, the insertion efficiency of the non-natural amino acid and the expression quantity of the non-natural amino acid protein can be increased.

## Description

### TECHNICAL FIELD

The present disclosure relates to the field of biotechnology, and preferably to an *in-vitro* cell-free protein synthesis system for inserting a non-natural amino acid.

### BACKGROUND

Proteins are important molecules in cells and are involved in the execution of almost all functions of cells. The different sequences and structures of the proteins determine the different functions thereof. Within a cell, protein can catalyze various biochemical reactions as enzymes, can coordinate various activities of an organism as signal molecules, and can support biological morphology, store energy, transport molecules, and move the organism. In the field of biomedicine, protein antibodies, as targeted drugs, are an important means of treating diseases such as cancer.

In cells, the production of proteins includes two parts: gene transcription and mRNA translation.

Gene transcription refers to a process of synthesizing RNA with 4 NTPs (ATP, CTP, GTP and UTP) as starting materials according to the complementary base pairing principle using one strand of DNA as a template under catalysis of DNA-dependent RNA polymerase (RNP or RNAP). For some RNA viruses, RNA can also guide the synthesis of RNA.

The translation of mRNA into protein refers to the assembly process of activated amino acids into protein polypeptide chains on ribosomes using mRNA as a template and tRNA as a carrier under the action of relevant enzymes and cofactors.

The regulation of protein synthesis plays an important role in dealing with external stresses such as nutrient deficiency, cell development and differentiation, and many other processes, including transcriptional regulation and translational regulation.

Transcriptional regulation refers to the regulation of RNA synthesis using DNA as a template. All cells have a large number of sequence-specific DNA binding proteins (trans-acting factors), and these proteins can accurately recognize and bind to specific DNA sequences (cis-acting elements), and play a role as a switch at the transcriptional level. Transcription-level regulation is an important part of eukaryotic gene expression regulation. According to whether eukaryotic gene expression is affected by the environment, eukaryotic gene expression can be classified into developmental regulation and transient regulation. The developmental regulation refers to the regulation of gene expression according to "predetermined" and "ordered" procedures to ensure the growth, development, differentiation and the like of eukaryotes, and is an irreversible process; the transient regulation refers to the adaptive transcriptional regulation performed by eukaryotes under the stimulation of internal and external environments, and is a reversible process.

Four processes of translational regulation include translation initiation, translation elongation, translation termination, and ribosome recycling, among which translation initiation is the most regulated process. Small ribosomal subunit (40S) binds to (tRNA) iMet in the beginning of translation, and recognizes the 5' end of mRNA under the action of a translation initiation factor. The small subunit moves downstream and binds to the large ribosomal subunit (60S) at the position of the start codon (AUG) to form a complete ribosome, which enters the translation elongation stage. Currently, commonly used biosynthetic systems are *in vivo* biosynthetic systems and *in vitro* biosynthetic systems. The *in vivo* biosynthetic system refers to a general term for the synthesis process of various compounds catalyzed by enzymes in an *in vivo* biological system, i.e., the assimilation reactions in an organism, including photosynthesis, gluconeogenesis, and the biosynthesis of nucleotides, nucleic acids, and proteins. Protein synthesis is quantitatively most important in cellular biosynthesis. Protein biosynthesis is also known as translation, a process of converting the arrangement order of bases in an mRNA molecule into the arrangement order of amino acids in a protein or polypeptide chain. Protein biosynthesis is divided into five stages: activation of amino acids, initiation of polypeptide chain synthesis, elongation of peptide chains, termination and release of peptide chains, and processing and modification after protein synthesis.

The *in vitro* biosynthesis system refers to the *in vitro* efficient synthesis of a specific chemical molecule or biological macromolecule (DNA, RNA, and protein ) in a lysis system of bacteria, fungi, plant cells or animal cells by adding exogenously encoded nucleic acid DNA, RNA, substrates and energy sources. A common *in vitro* biosynthesis system is the *in vitro* protein synthesis system, i.e., a cell-free protein synthesis system, which completes the rapid and efficient translation of exogenous recombinant proteins by means of an exogenous mRNA or DNA template and the use of cell lysates.

Cell-free systems can be traced back to Buchner who proposed in 1897 that biosynthesis can be carried out *in vitro.* He demonstrated the production of bioethanol by the yeast cell-free system. However, due to adenosine triphosphate (ATP) imbalance, the system is not suitable for large-scale applications. Welch and Scopes solved the above problems through various explorations in 1985 and obtained a high yield of ethanol, but the system also has two major defects: the need to add additional high-cost enzymes and the intolerance to temperature changes.

However, at present, the technology has some inherent problems that are difficult to solve, such as reversibility, instability, leakage, inactivation, cyclic use of enzymes, lack of stable enzymes, enzyme complexes, and cofactors.

A commercially common *in vitro* protein synthesis system is an *in vitro* transcription-translation coupling system (*in vitro* transcription-translation system, abbreviated as IVTT): an mRNA intermediate is obtained by transcription from a DNA template with RNA polymerase, and then amino acids, ATP and other components are utilized to complete one-step efficient translation of exogenous proteins. At present, common commercial *in vitro* protein expression systems include *Escherichia coli* extract (ECE) system, Rabbit reticuLocyte lysate (RRL) system, wheat germ extract (WGE) system, insect cell extract (ICE) system and human system.

Compared with the traditional *in vivo* recombinant expression system, the *in-vitro* cell-free protein synthesis system has various advantages. For example, it can express special proteins that are toxic to cells or contain non-natural amino acids (such as D-amino acids), and can synthesize a variety of proteins in parallel directly using the PCR product as a template for high-throughput drug screening and proteomics research.

As an important site for post-translational modification of proteins and a key residue in the activity center of various enzymes, non-natural amino acids play an important role in the process of various proteins performing their physiological and pathological functions. For proteins, particularly polypeptide drugs, the modification of non-natural amino acids may not only enhance the efficacy of the polypeptide drugs and reduce the toxicity of the drugs, but also greatly reduce the immunogenicity of the polypeptide drugs and reduce the immune rejection due to the incorporation of non-natural amino acids, and some proteases may no longer recognize the polypeptide drugs incorporating non-natural amino acids, so that the drugs are not degraded for a longer time in the body, thereby prolonging the half-life of the drugs and eliminating the disadvantages of continuous injection administration of polypeptide drugs. Moreover, it is also expected that the polypeptide drugs "carry" other chemical accessories through modification, thereby leading to the emergence of new methods for treating diseases.

The post-translational modification of proteins by site-directed modification of non-natural amino acids is of great significance for the synthesis of special polypeptides or proteins, the influence of chemically modified amino acids on protein structure and function, and the like.

At present, the relatively mature technology for the artificial synthesis of protein substances with site-directed modification of non-natural amino acids is the chemical polypeptide synthesis technology, which can achieve the synthesis of some short fragments by liquid phase or solid phase synthesis. However, such methods have obvious limitations. Liquid phase synthesis mainly relies on the spontaneous coupling of amino acids in the reaction system, so the efficiency cannot be guaranteed. It is difficult to obtain pure polypeptide products as to finally separate the starting materials, activators, etc. in the reaction system.

### SUMMARY

The object of the present disclosure is to provide a reaction system, a kit and a reaction method for improving the synthesis efficiency of proteins containing a non-natural amino acid.

In a first aspect, the present disclosure provides an *in-vitro* cell-free protein synthesis system for inserting a non-natural amino acid, wherein the reaction system comprises:
(1) a cell extract;
(2) a non-natural amino acid;
(3) an exogenous orthogonal aminoacyl tRNA synthetase/orthogonal tRNA pair;
(4) a template comprising a target protein gene sequence, wherein at least one codon encoding an amino acid in the target protein gene sequence is mutated into a stop codon.

In another preferred embodiment, the stop codon is mutated into TAG.

In another preferred embodiment, a codon for one amino acid in the gene sequence of the target protein is mutated into a stop codon.

In another preferred embodiment, codons for two or more amino acids in the gene sequence of the target protein are mutated into a stop codon.

In another preferred embodiment, the cell extract is preferably selected from any one of the following sources: *Escherichia coli,* a yeast cell, a mammalian cell, a plant cell, an insect cell, and combinations thereof.

In another preferred embodiment, the cell extract is more preferably selected from any one of the following sources: *Escherichia coli, K. lactis,* wheat germ cells, Spodoptera frugiperda insect cells, rabbit reticulocytes, CHO cells, COS cells, VERO cells, BHK cells, human fibrosarcoma HT1080 cells, and combinations thereof.

In another preferred embodiment, the yeast cell is preferably selected from *Pichia pastoris, Pichia finlandica, Pichia trehalophila, Pichia koclamae, Pichia membranaefaciens, Pichia minuta* (methanol-inducible yeast (*Ogataea minuta*) and *Pichia lindneri*)*, Pichia opuntiae, Pichia thermotolerans, Pichia salictaria, Pichia g uercuum, Pichia pijperi, Pichia stiptis, Pichia methanolica, Pichia sp., Saccharomyces cerevisiae, Saccharomyces sp., Hansenula polymorpha, Kluyveromyces, K. lactis, Kluyveromyces marxianus,* Kluyveromyces marxianus var.lactis, Kluyveromyces marxianus var.marxianus, *Kluyveromyces marxianus var.vanudenii, Kluyveromyces dobzhanskii, Kluyveromyces aestuarii, Kluyveromyces nonfermentans, Kluyveromyces wickerhamii, Kluyveromyces thermotolerans, Kluyveromyces fragilis, Kluyveromyces hubeiensis, Kluyveromyces polysporus, Kluyveromyces siamensis,* and *Kluyveromyces yarrowii,* and combinations thereof. In another preferred embodiment, the *Kluyveromyces* yeast is more preferably selected from *Kluyveromyces marxianus* and/or *K. lactis.*

In another preferred embodiment, the yeast cell extract is an aqueous extract of yeast cells.

In another preferred embodiment, the yeast cell extract is free of long-chain nucleic acid molecules endogenous to yeast.

In another preferred embodiment, the yeast cell extract is prepared by a method comprising:
(i) providing a yeast cell;
(ii) washing the yeast cell to obtain a washed yeast cell;
(iii) subjecting the washed yeast cell to cell disruption treatment to obtain a crude yeast extract; and
(iv) subjecting the crude yeast extract to solid-liquid separation to obtain a liquid fraction, i.e., the yeast cell extract.

In another preferred embodiment, the centrifugation is performed in a liquid state.

In another preferred embodiment, the centrifugation is performed at 5000-100000 g, preferably 8000-30000 g.

In another preferred embodiment, the centrifugation is performed for 0.5 min to 2 h, and preferably 20-50 min.

In another preferred embodiment, the centrifugation is performed at 1-10 °C, preferably 2-6 °C.

In another preferred embodiment, the washing is performed using a washing solution at a pH of 7-8 (preferably 7.4).

In another preferred embodiment, the washing solution is selected from: potassium 4-hydroxyethylpiperazine ethanesulfonate, potassium acetate, magnesium acetate, and combinations thereof.

In another preferred embodiment, the cell disruption treatment comprises high-pressure disruption and freeze-thaw (such as liquid nitrogen low-temperature) disruption.

In another preferred embodiment, a structural formula of the non-natural amino acid is a compound of formula (I), 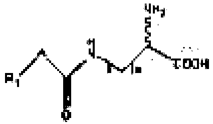 wherein n is selected from natural numbers of 1-20, R₁ is selected from substituted or unsubstituted C5-C60 aryl or heteroaryl, substituted or unsubstituted C1-C20 alkyl, substituted or unsubstituted C2-C20 alkenyl, and substituted or unsubstituted C2-C20 alkynyl, and A is selected from O and -CH₂-.

In another preferred embodiment, n is selected from 1, 2, 3, 4, 5, 6, 7, 8, 9, 10, 11, 12, 13, 14, 15, 16, 17, 18, 19, and 20.

In another preferred embodiment, n is selected from natural numbers of 1-10.

In another preferred embodiment, n is selected from 1, 2, 3, 4, 5, 6, 7, 8, 9, and 10.

In another preferred embodiment, n is selected from natural numbers of 1-6.

In another preferred embodiment, n is selected from 1, 2, 3, 4, 5, and 6.

In another preferred embodiment, R 1 is selected from substituted or unsubstituted C5-C30 aryl and heteroaryl.

In another preferred embodiment, R 1 is selected from substituted or unsubstituted phenyl.

In another preferred embodiment, R 1 is selected from substituted or unsubstituted C2-C20 alkenyl.

In another preferred embodiment, R 1 is selected from substituted or unsubstituted C2-C10 alkenyl.

In another preferred embodiment, R 1 is selected from substituted or unsubstituted C2-C6 alkenyl.

In another preferred embodiment, R 1 is selected from substituted or unsubstituted C2-C20 alkynyl.

In another preferred embodiment, R 1 is selected from substituted or unsubstituted C2-C10 alkynyl.

In another preferred embodiment, R 1 is selected from substituted or unsubstituted C2-C6 alkynyl.

In another preferred embodiment, A is selected from O.

In another preferred embodiment, A is selected from -CH₂-.

In another preferred embodiment, the substituent is a substituent group commonly used in the art, such as aryl, heteroaryl, alkyl, cycloalkyl, aryloxy, heteroaryloxy, alkyloxy, cycloalkyloxy, hydroxy, mercapto, ester group, carboxy, cyano, halogen, nitro, sulfonic acid group, azido, alkenyl, alkynyl, phosphoric acid group, and the like.

In another preferred embodiment, the structural formula of the non-natural amino acid is selected from one and combinations of the following: 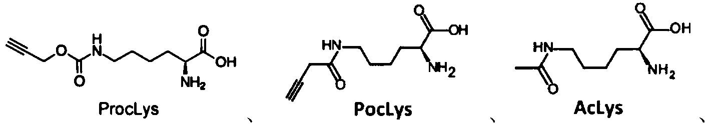 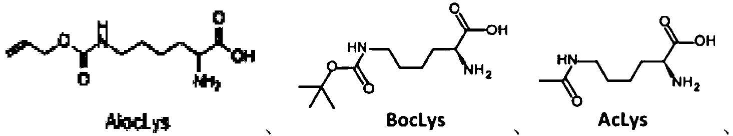 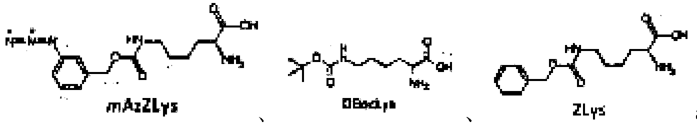 and

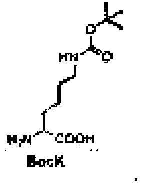

In another preferred embodiment, the relative concentration of the non-natural amino acid to the reaction system is in a range of 0.1-1000 mmol/L, preferably 0.5-500 mmol/L, and more preferably 5-100 mmol/L.

In another preferred embodiment, the exogenous orthogonal aminoacyl tRNA synthetase is selected from natural or mutated Pyl-tRNA synthetase (PylRS), Leu-tRNA synthetase (LeuRS), Tyr-tRNA synthetase (TyrRS), Phe-tRNA synthetase (PheRS) and TrP-tRNA synthetase (TrpRS); the tRNA is selected from natural or mutated tRNAPyl, tRNALeu, tRNATyr, tRNAPhe and tRNATrp.

In another preferred embodiment, the exogenous orthogonal aminoacyl tRNA synthetase is selected from natural or mutated MaPylRS, MmPylRS, MbPylRS, EcTyrRS, MjTyrRS, EcLeuRS, ScPheRS, ScTrpRS, and BsTrpRS, preferably MaPylRS.

In another preferred embodiment, the relative concentration of the enzyme to the reaction system is in a range of 0.001-1 mmol/L, preferably 0.005-0.1 mmol/L, and more preferably 0.005-0.05 mmol/L.

In another preferred embodiment, the relative concentration of the tRNA to the reaction system is in a range of 0.001-1 mmol/L, preferably 0.005-0.1 mmol/L, and more preferably 0.02-0.1 mmol/L.

In another preferred embodiment, the target protein is selected from fluorescein protein, luciferase, green fluorescent protein, yellow fluorescent protein, red fluorescent protein, glyceraldehyde-3-phosphate dehydrogenase, catalase, actin, a variable region of an antibody, a luciferase mutation, α-amylase, enterocin A, hepatitis C virus E2 glycoprotein, insulin precursor, interferon αA, interleukin-1β, lysozyme, serum albumin, single-chain variable fragment (scFV), transthyretin, tyrosinase, xylanase, and combinations thereof.

In another preferred embodiment, the system further comprises: one or more of a buffer, potassium ion, magnesium ion, polyethylene glycol, an optional aqueous solvent, and a phosphate.

In another preferred embodiment, the buffer is selected from: one of Tris-HCl, Tris base, HEPES, Tris-citric acid, citric acid-citrate, and Tris-citrate, and combinations thereof.

In another preferred embodiment, the potassium ion is derived from a potassium ion source, which is not particularly limited and is selected from: one of potassium acetate, potassium glutamate, and potassium citrate, and combinations thereof.

In another preferred embodiment, the potassium ion concentration is 30-210 mM, preferably 30-150 mM, and more preferably 30-80 mM.

In another preferred embodiment, the magnesium ion is derived from a magnesium ion source, which is not particularly limited and is selected from: one of magnesium acetate, magnesium glutamate, magnesium citrate and magnesium aspartate, and combinations thereof.

In another preferred embodiment, the polyethylene glycol is selected from: PEG3000, PEG8000, PEG6000, PEG3350, and combinations thereof.

In another preferred embodiment, the phosphate is selected from orthophosphate, monobasic phosphate, dibasic phosphate, metaphosphate, pyrophosphate, and combinations thereof, preferably orthophosphate.

In another preferred embodiment, the concentration v/v of the cell extract is 20%-80%.

In another preferred embodiment, the concentration (w/v) of the polyethylene glycol is 0.1%-8%, preferably 0.5%-4%, and more preferably 1%-2%.

In a second aspect, the present disclosure provides a kit comprising (a) a container, and (b) the synthesis system according to any one of the first aspect of the present disclosure in the container.

In a third aspect, the present disclosure provides a method for synthesizing a protein comprising a non-natural amino acid using a cell-free system, wherein the preparation is performed using the synthesis system according to any one of the first aspect of the present disclosure or the reagent kit according to the second aspect of the present disclosure.

In a fourth aspect, the present disclosure provides use of the system according to the first aspect of the present disclosure or the kit according to the second aspect of the present disclosure in the synthesis of a protein comprising a non-natural amino acid.

In a fifth aspect, the present disclosure provides a protein comprising a non-natural amino acid, prepared by the synthesis system according to any one of the first aspect of the present disclosure or the kit according to the second aspect of the present disclosure or the method according to the third aspect of the present disclosure.

In a sixth aspect, the present disclosure provides use of the protein comprising the non-natural amino acid provided in the fifth aspect of the present disclosure in click chemistry modification.

It will be appreciated that within the scope of the present disclosure, the above technical features of the present disclosure and the technical features specifically described below (as in the examples) can be combined with each other to constitute a new or preferred technical solution. Due to limited space, such schemes are not described herein.

### BRIEF DESCRIPTION OF THE DRAWINGS

FIG. 1 shows a schematic diagram of the construction of MaPylRSpET28a plasmid constructed in the present disclosure.
FIG. 2 shows a schematic diagram of pET28a-tRNA ^{Pyl}_{CUA} plasmid constructed in the present disclosure.
FIG. 3 shows the constructed single-site incorporation reporter gene.
FIG. 4 shows a schematic diagram of the dual-fluorescent reporter gene related to the present disclosure. The purpose is to detect the efficiency and authenticity of the insertion of non-natural amino acids. OTS: orthogonal translation system, representing a biologically orthogonal protein translation system; ncAA: noncanonical amino acid, representing a non-natural amino acid; F: representing a natural amino acid Phe; X: noncanonical amino acid, representing a non-natural amino acid.
FIG. 5 shows Proteinfactory non-natural amino acid insertion reporter gene of the present disclosure and different OTS ingredients, wherein the expression supernatant is purified by a nickel column. NC indicates ncaa only, PC indicates a positive control, ETC on the left indicates containing ncaa reporter gene + OTS, and ET indicates containing ncaa reporter gene + o-aars + o-tRNA, and EC represents ncaa reporter gene + O-aars + ncaa, and ETC on the right represents the reused ncaa reporter gene + OTS (o-aars + o-tRNA + ncaa).
FIG. 6.1 shows the estimation of target protein expression level with non-natural amino acids based on proteinfactory-OTS.
FIG. 6.2 shows that after the target protein is purified, SDS-PAGE electrophoresis is performed, and the expression level is estimated using ImageJ software. The grayscale value of the eGFP-ncaa-scarlet protein band is about 0.357 times that of the input O-aaRs. The concentration of the added O-aaRs is known to be 1.2 mg/mL. Therefore, the expression level of eGFP-ncaa-scarlet protein can be calculated to be about 0.43 mg/mL.
FIG. 7 shows the mass spectrometry analysis results of the protein with the non-natural amino acid label, the mass spectrometry analysis results being: The relative content of the protein with the non-natural amino acid label is 99.81%.
FIG. 8 shows the fluorescence values of purified proteins labeled with non-natural amino acids.
FIG. 9 shows the concentration (mg/mL) of the purified protein presumed from the BCA standard curve, and the total amount of the expressed protein is reversely calculated from the fluorescence values of the purified proteins.

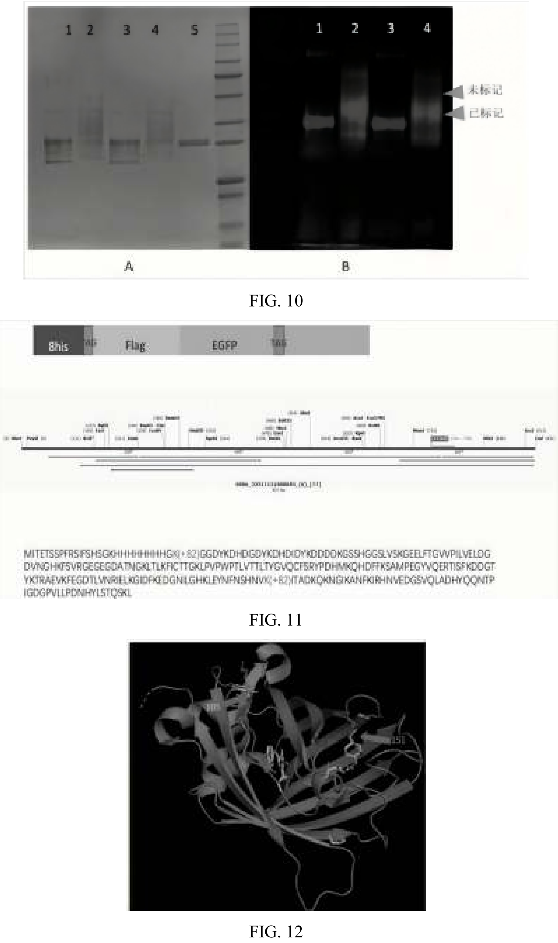

FIG. 10 shows the detection results of labeling the reporter gene inserted with POCK by click chemistry reaction. The purified reporter gene protein is subjected to click chemistry experiment to react with azide-CY5, and the fluorescent picture shows that the target protein is fluorescently labeled with red fluorescene after the reaction. A is a Coomassie-stained photograph, B is a fluorescent photograph, 1 and 3 denote the denatured sample after labeling, 2 and 4 denote the non-denatured sample after labeling, and 5 denotes the denatured sample before labeling. Red for cy5 protein (610 nm), and blue for GFP protein (435 nm).
FIG. 11 shows the constructed ncaa double-site insertion gene of the present disclosure, in which the codon of Y at position 151 of GFP is mutated into TAG to detect the insertion of non-natural amino acids. The first insertion site is located between two tags (8*Histag) and (3*Flag), which is located at the N terminus of the whole GFP protein, and has less influence on the structure and fluorescence intensity. The second insertion site is 151 Try. According to the structure of EGFP, 151Tyr is located at the egress of the barrel structure, which is presumed to have less influence on the structure. Sequencing results indicate that the mutation is as expected.
FIG. 12 shows the structure and effect of the synthesized ncaa double-site insertion protein of the present disclosure.
FIG. 13 shows the ncaa triple-site insertion gene constructed by the present disclosure, with K at position 2 selected for the third insertion site. The insertion site of the third non-natural amino acid is selected as 105Tyr, which is also located outside the EGFP barrel result and does not affect the
fluorescence characteristics. Sequencing results prove that the mutation is correct and meets the expected design.

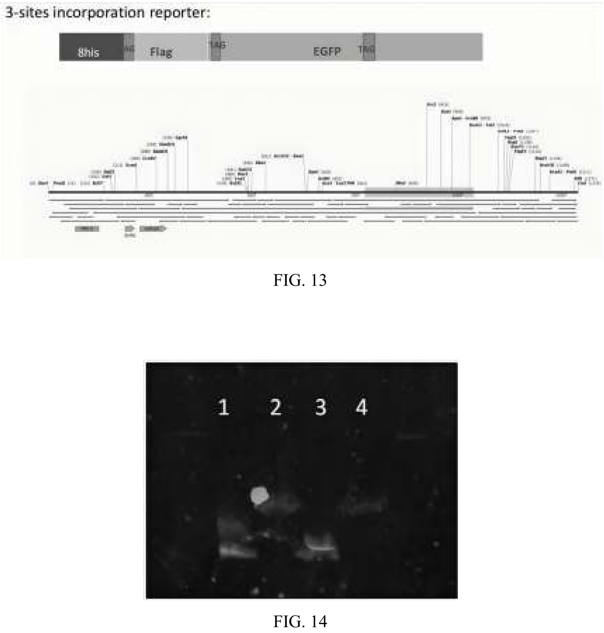

FIG. 14 shows a fluorescence image of the protein synthesized by the ncaa triple-site insertion gene. 1 is an expression supernatant (MW = 35 KDa) of GFP gene with triple-site TAG (3 sites) in a cell-free *in vitro* orthogonal translation system. 2 is the expression supernatant (MW = 58.5 KDa) fused with a TEV enzyme-TEV enzyme cutting site (with a TAG mutation)-EGFP gene. The construct has an insertion site for a non-natural amino acid, and GFP can be expressed only when the non-natural amino group is inserted into a synthetic polypeptide chain, which has green fluorescence. Also, the TEV enzyme cutting site has non-natural amino acid mutation and therefore cannot be recognized by TEV. The molecular weight of the fusion protein is 58.5 KDa. 3 is a sample of 1 purified by a Ni column. 4 is a sample of 2 purified by a Ni column. From this, it can be seen that the results show that the GFP gene with 3 TAG stop codons can also be expressed in a cell-free *in vitro* orthogonal translation system. The fusion protein with a TEV label also indicates that TAG can introduce non-natural amino acids into the polypeptide chain of a protein.

### DETAILED DESCRIPTION

After an extensive and intensive research, through extensive screening and exploration, a reaction system capable of improving the synthesis efficiency of proteins containing non-natural amino acids has been unexpectedly found for the first time. In an *in vitro* synthesis system for *K. lactis* cells, a standard protein (eGFP) has a single-site non-natural amino acid (POCK, or Pock, or pock, i.e., a compound Proclys) insertion efficiency of 99.81%, and the expression level of the protein of the target protein after the incorporation of nacc can reach 0.43 mg/mL. In addition, it can be further confirmed through experiments that proteins containing non-natural amino acids are capable of performing click chemistry modification.

The present disclosure is intended to establish an *in vitro* translation and site-directed modification system for protein based on a eukaryotic cell. A basic technical approach is to add a biologically orthogonal translation system (OTS) in a ProteinFactory system. OTS contains non-classical aminoacyl tRNA synthetase (O-aaRs) with non-natural amino acids (noncanonical amino acid, ncaa) as substrates, and O-tRNA recognized by ncaaRS. O-tRNA binds to and is biologically orthogonal to cellular natural aminoacyl tRNA synthetase and cannot be used as a substrate for aminoacylation.

O-aaRs can specifically catalyze the aminoacylation reaction of o-tRNA and ncaa. Generally, the anticodon of o-tRNA is engineered to be paired with and bind to an amber stop codon (TAG), such that the naturally terminated mRNA signal is recognized and translated into non-natural amino acids. The chemically active groups of these non-natural amino acids can further form covalent bonds with probe molecules or other molecules by click chemistry to achieve specific modification of the target protein.

In the following examples, 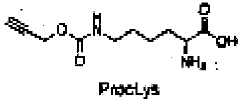 (i.e., POCK) is selected as a representative of ncaa. However, it is not limited that ncaa in the present application only refers to POCK.

"Expression system of the present disclosure", *"in-vitro* expression system of the present disclosure", and *"in vitro* cell-free expression system" are used interchangeably and all refer to the *in-vitro* protein expression system of the present disclosure, and other descriptions may also be used, such as: a protein *in vitro* synthesis system, an *in vitro* protein synthesis system, a cell-free system, a cell-free protein synthesis system, a cell-free *in vitro* protein synthesis system, an *in-vitro* cell-free protein synthesis system, an *in-vitro* cell-free synthesis system, a CFS system (cell-free system), a CFPS system (cell-free protein synthesis system), and the like. According to the reaction mechanism, it may comprise an *in vitro* translation system (abbreviated as IVT system, an mR2P system), an *in vitro* transcription and translation system (abbreviated as IVTT system, a D2P system), an *in vitro* replication, transcription and translation system (abbreviated as IVDTT system, a D2P system), etc. In the present disclosure, an IVTT system is preferred. We have also termed the *in vitro* protein synthesis system a "Protein Factory" or "protein factory" or "proteinfactory". The components of the *in vitro* protein synthesis system provided herein are described in an open-ended manner.

The final concentrations of the components in the ProteinFactory described herein are as follows: 80% (v/v) *K. lactis* extract, 15 mM glucose, 320 mM maltodextrin (molar concentration based on glucose monomer), 24 mM tripotassium phosphate, 1.8 mM nucleoside triphosphate mixture (adenine nucleoside triphosphate, guanosine nucleoside triphosphate, cytosine nucleoside triphosphate, and uridine nucleoside triphosphate, each at a final concentration of 1.8 mM), 0.7 mM amino acid mixture (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine, each at a final concentration of 0.7 mM), magnesium L-aspartate, 80 mM potassium acetate, 2% (w/v) polyethylene glycol 8000, 9.78 mM pH 8.0 Tris·HCl buffer, and 6% (w/v) trehalose. The *K. lactis* extract comprises an endogenously expressed T7 RNA polymerase. The preparation of the *K. lactis* cell extract was carried out by conventional technical means. Reference is made to CN109593656A. In general, the preparation steps comprise: providing an appropriate amount of starting material for *K. lactis* cells subjected to fermentation culture, rapidly freezing the cells with liquid nitrogen, breaking the cells, and centrifuging to collect a supernatant to obtain the cell extract. The protein concentration in the obtained *K. lactis* cell extract was 20-40 mg/mL.

### Example 1: Purification of MaPylRs Protein

### 1.1 Construction of MaPylRSpET28a plasmid

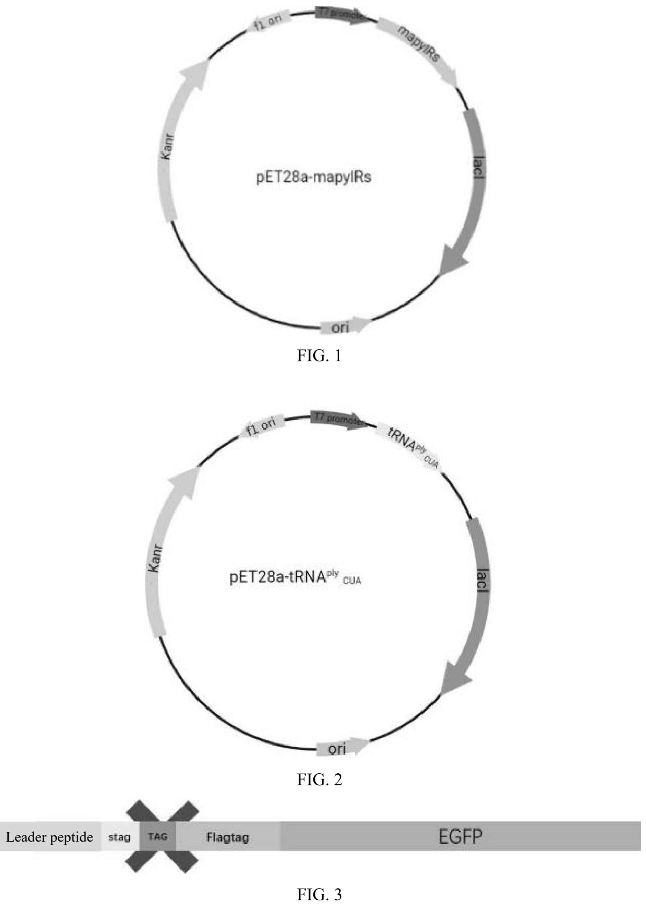

The gene from Candidatus Methanomethylophilusalvus (PylRSs (pyrrolysyl tRNA synthetase ) with gene sequence number of WP_015505008) was subjected to gene codon usage bias optimization, synthesized (Sangon) and cloned into an *Escherichia coli* expression vector pET28a vector NcoI/BamHI site (see FIG. 1).

1.2 Induced expression of MaPyLRs: pET28a-MaPylRs plasmid was transformed into *E. coli* competent cell BL (DE3) cells.

Monoclonal cells were picked, cultured overnight in 100 mL of LB medium containing 50 mg/mL kanamycin, and subjected to expansion culture. 1000 µL of the above culture solution was added to 1 L LB medium containing 50 mg/mL kanamycin, and cultured overnight at 37 °C. When the bacterial density OD600 = 0.6, IPTD at a final concentration of 0.2 mM was added, and the mixture was transferred to 16 °C and cultured for another 20 h.

1.3 MaPyLRs purification: The bacteria after the induced expression were collected by centrifugation, crushed by a high-pressure homogenizer, and centrifuged at 12000 rpm to remove the precipitate, and the supernatant was purified by HisTrpFF (GE). Equilibration buffer
Buffer A: 25 mM TrisHCL (pH 7.6), 20 mM imidazole, 5% glycerol, and elution buffer
Buffer B: 25mM TrisHCL (pH7.6), 250 mM imidazole, 5% glycerol. Gradient elution was followed the usual purification procedure.

1.4 MaPyLRs concentration: The eluted proteins were combined and concentrated using an ultrafiltration centrifugal concentration tube (millipore). After concentration, the sample was dialyzed against Buffer A overnight.

1.5 The protein concentration was quantified by the BCA method.

### Resulting MaPylRS

### Example 2: tRNA ^{Pyl}_{CUA} In Vitro Transcription

### 2.1 Construction of pET28a-tRNA ^{Pyl}_{CUA} plasmid

tRNA ^{pyl}_{CUA} gene sequence: Genebank number: CP017686.1 The target fragment was amplified by PCR, GGGGGACGGTCCGGCGACCAGCGGGTCTCTAAAACCTAGCcAGCGG GGTTCGACACCCCGGTCTCTCGcca (SEQ ID No: 2) and linked to PET28a vector downstream of T7 promoter (see FIG. 2).

### Example 3: Construction of Single-Site Incorporation of Reporter Gene

With pD2P8His-EGFP as a template, first, a stop codon TAG and a Flag tag were inserted between the 8His tag and the reporter gene by PCR method; and in the second part, the 8His tag in the original template was replaced with the Stag tag. To stabilize the stability of the reporter gene, a reporter gene as shown in FIG. 3 was finally constructed.

### Primer:

Insertion of TAG-Flag tag:
Primer₁: ACCACCACCACGGTTAGGGTGGGGACTACAAGGATCACGACG (SEQ ID No: 3)
Primer2: ctccatggctGGATCCCTTATCGTCGTCATCCTTGTAATCG (SEQ ID No: 4)

Corresponding vector PCR primer:
primer3: TTGTAGTCCCCACCCTAACCGTGGTGGTGGTGGTG (SEQ ID No: 5)
Primer4: TACAAGGATGACGACGATAAGGGATCCagccatggaggaag (SEQ ID No: 6)

Insertion of stag tag and replacement by 8His tag:
Primer 5: ACTCTGGTAAGaaggaaaccgctgctgctaaattcgaacgccagc (SEQ ID No: 7) Primer 6: vector PCR Primer corresponding to CCCCACCCTAACCgctgtccatgtgctggcgttcgaatttagcagc (SEQ ID No: 8):
Primer7: cagcggtttccttCTTACCAGAGTGAGAGAAGATAGATCT GAATGG (SEQ ID No: 9)
Primer8: cgccagcacatggacagcGGTTAGGGTGGGGACTACAAGGATCAC (SEQ ID No: 10)

The finally obtained reporter gene had a sequence of SEQ ID NO: 11.

### Example 4: Insertion of Non-Natural Amino Acids (Pock) into EGFP Polypeptide Chain

### Cell-free in vitro translation conditions:

ProteinFactory was dissolved by ddH2O.
1 mL of proteinfactory
10 µL of 500 mM pock (final concentration of 5 mM)
20 µM MaPylRs (final concentration)
20 µM tRNA ^{pyl}_{CUA} (final concentration)
30 µL of target gene template PCR product
28 °C, overnight reaction

The target protein was obtained.

### Example 5: Detection of Insertion Efficiency and Authenticity of Non-Natural Amino Acids

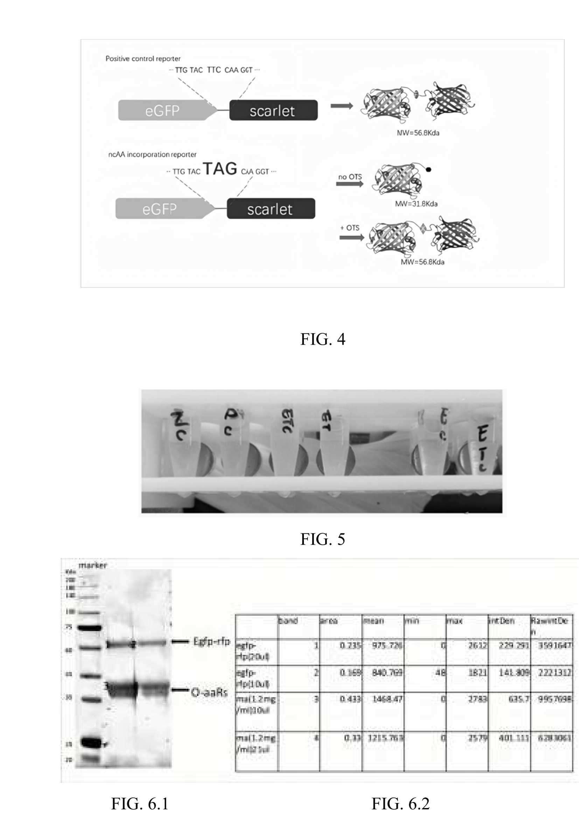

To detect the insertion efficiency and authenticity of non-natural amino acids, we designed two dual-fluorescent reporter genes (FIG. 4).

Readthrough efficiencies for stop codons for non-natural amino acid introduction were denoted as RRE (relative readthrough efficiency), wherein: rfp (TAG+ncaa) and rfp (positive control) represented the expression levels of the TAG reporter gene added with the orthogonal translation system and the positive control group (natural amino acids) C-terminal fluorescent protein, respectively; gfp (TAG+ncaa) and gfp (positive control) represented the expression levels of the TAG reporter gene added with the orthogonal translation system and the positive control group (natural amino acids) N-terminal fluorescent protein, respectively.

The closer RRE is to 1, the closer the efficiency of insertion of the non-natural amino acid is to the natural amino group. The natural amino acid error introduction frequency MMF (maxium mistranslation frequency) of the target site in the product was shown.

Accordingly, the proportion of the non-natural amino acids at this site is 1-MMF.

Ideally, the expression of the C-terminus fluorescent protein is zero in the absence of non-natural amino acids, i.e., RRE (TAG-ncaa) = 0, so that the closer the MMF is to 0, the higher the introduction proportion of non-natural amino acids is, and only the expected insertion of non-natural amino acids at specific sites of the target protein was observed.

### Experimental results:

Adding OTS to proteinfactory realized readthrough of stop codon
1, and only after the addition of the intact OTS, the scarlet (red) behind the codon of ncaa-reporter TAG could be successfully translated and expressed, as shown in FIG. 5. The cell supernatant turned from green to red (ETC sample in FIG. 5), so this process was specific.

Estimation of target protein expression level with non-natural amino acids was performed based on proteinfactory-OTS (FIG. 6.1). After the target protein was purified, SDS-PAGE electrophoresis was performed, and the expression level was estimated using ImageJ software (FIG. 6.2). The grayscale value of the eGFP-ncaa-scarlet protein band was about 0.357 times that of the input O-aaRs. The concentration of the added O-aaRs is known to be 1.2 mg/mL. Therefore, the expression level of eGFP-ncaa-scarlet protein was about 0.43 mg/mL.

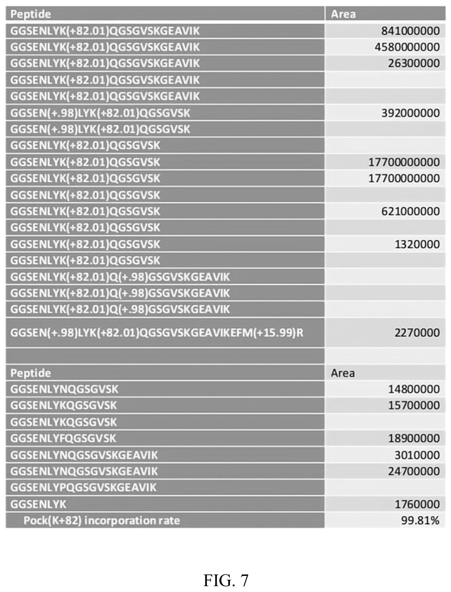

For the mass spectrometry analysis results of the protein with the non-natural amino acid label, the mass spectrometry analysis results were: The relative content of the protein with the non-natural amino acid label was 99.81% (FIG. 7).

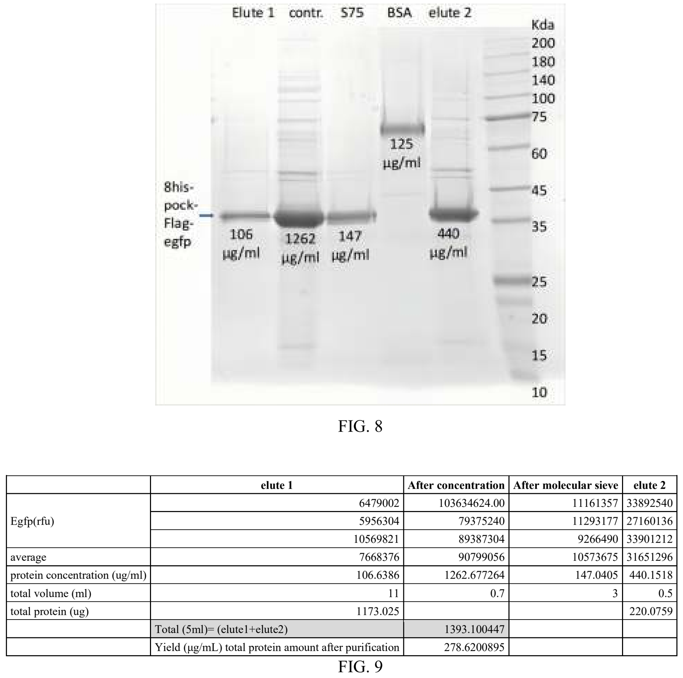

After protein purification, the yield of the non-natural amino acid incorporated protein was measured to be 278 µg/mL by the BCA method (FIGs. 8 and 9).

Example 5: Use of Proteins Comprising Non-Natural Amino Acids in Click Chemistry Modification
(I) Preparation of stock solution:
   1, 20 mM CuSO4:31 mg CuSO4 was dissolved in 10 mL of sterile ddH2O, and the mixture was aliquoted and stored at -20 °C;
   2, 50 mM THPTA:1 mg THPTA (cas760952-88-3sigma) was dissolved in 46 µL ddH2O and stored at -20 °C;
   3, 100 mM BTTAA:4.3 mg BTTAAHY-100486MCE was dissolved in 100 µL of DMSO, and stored at -20 °C;
   4, 1 mM Cy5-azide and 1 mg Cy5-azide (sigma-aldrich777323) were dissolved in 1 mL of DMSO, and maintained in the dark at -20 °C;
   5, 100 mM aminoguaidin (CAS number: 1937-19-5 Sigma-Aldrich) 0.11 g aminoguidin was dissolved in 10 mL of ddH2O, and the mixture was aliquoted and stored at -20 °C.
   6, 100 mM sodium L-ascorbate (sigma-Aldirich A7631) 0.198 g was dissolved in 10 mL of ddH2O, and the mixture was aliquoted and stored at -20 °C.
(II) Experimental method I:
   1, a CuSO4-THPTA premixed solution was prepared:
      10 µL of 20 mM CuSO4
      20 µL of 50 mM THPTA mixed
   2, a reaction solution was prepared:
      200 µL of purified protein with non-natural amino acid at a concentration of ≈ 30 µM
      120 µL of 1 mM cy5-azide
      9 µL of CuSO4-THPTA premixed solution
      30 µL of 100 mM Aminoguaidin (cas1937-19-5 Innochem)
      220 µL of PBS
      30 µL of 100 m sodium L-ascorbate (cas134-03-2 Sangon)

The above were added in sequence, gently mixed, and then sealed.

The mixture was placed on a mixer and reacted overnight at 4 °C in the dark.

### (III) Verification

The purified protein containing non-natural amino acids was subjected to click chemistry experiment to react with cy5-azide. Fluorescent photographs showed that the target protein was fluorescently labeled with red fluorescene after the reaction. However, the non-denatured sample showed that some proteins were not labeled (see FIG. 10). This indicated that the protein containing non-natural amino acids can be chemically modified by reacting with cy5-azide through click chemistry.

### Example 6:

Construction of Double-Site Incorporation of Reporter Gene
Primer 9: TAG2F: (SEQ ID No: 12)
   ACCACCACCACCACGGTTAGGGTGGGGACTACAAGGATCACG
Primer 10: TAG2R: (SEQ ID No: 13)
   TCCTTGTAGTCCCCACCCTAACCGTGGTGGTGGTGGTGGTG
Primer 11: (SEQ ID No: 14)
   TAG151F:AACTCTCACAACGTTTAGATCACCGCTGACAAGCAAAAGAACG
Primer 12: TAG151R: (SEQ ID No: 15)
   TGTCAGCGGTGATCTAAACGTTGTGAGAGTTGAAGTTGTATTCC

The DNA sequence (SEQ ID NO: 16) of pD2PeGFP was used as a template for PCR amplification. After ligation and transformation, single clones were picked for sequence identification.

First, a single-site was constructed to insert the reporter gene:
(1) PCR amplification was performed using a Phanta^{®} MaxSuper-Fidelity DNA Polymerase kit from Vazyme.
   PCR reaction system:
      2*phantaMax Buffer 25 µL
      dNTPmix (10 mM each) 1 µL
      DNA template 1 µL
      Primer 9 and primer 10 (10 µM) 2 µL for each
      PhantaMaxsuper-fidelity DNA polymerase 1 µL
      Supplement with ddH2O to a final volume of 50 µL
   PCR reaction procedure:
      pre-denaturation: 95 °C for 3 min;
      denaturation 95 °C for 15 s;
      annealing 60 °C for 15 s;
      extension 72 °C for 30 s/Kb
      35 cycles
      72 °C 5 min

The correctness of the molecular weight of the PCR product was detected by electrophoresis.
(2) PCR product ligation:
   10 µL of vector and the inserted PCR amplification product were mixed, and 1 µL of DpnI (NEBR0176S) was added.
   37 °C 15 min.
(3) Conversion of ligation product
   30 µL of competent cells DH5α (Weidi Bio DL1001) was added with 3 µL of ligation product, and the mixture was incubated in an ice bath for 30 min, incubated at 42 °C for 45 s, added with 200 µL of LB medium, and thawed at 37 °C for 1 h. The resulting mixture was applied on LB agar plates with 1 µg/mL ampicillin resistance.

The plates were cultured at 37 °C overnight. Single clones were picked and sent to Sangon Biotech for sequencing. The sequencing primers were T7 and T7 terminal. A template plasmid pD2P-8his-egfp10 (SEQ ID No: 17) was prepared.

Further, using the plasmid with the correct sequence described above as a template, PCR amplification was performed using primer 11 and primer 12:TAG151R according to the same method, followed by ligation and transformation into DH5α competent cells. Single clones were picked and sent to Sangon Biotech for sequencing. A reporter gene plasmid with correct sequence and double-site insertion was obtained.

### Example 7: Synthesis of Target Protein with Double-Site Incorporation of Reporter Gene

7.1 The plasmid template was amplified. After the bacteria verified by sequencing were cultured overnight, the plasmid was extracted using a mini plasmid extraction kit.

7.2 Amplification of target gene
Primer 13 PD2PF: GGTGATGTCGGCGATATAGGCGCC (SEQ ID No: 18) and primer 14 PD2PR: TGCTCAGCGGTGGCAGCAGCCAAC (SEQ ID No: 19)

After being amplified, the PCR product can be directly used for protein translation in a cell-free system without purification and concentration.

4.3 Non-natural amino acids (Pock) were inserted at two sites of the EGFP polypeptide chain in a cell-free system.

*In vitro* translation conditions:
ProteinFactory was dissolved by ddH2O.
1 mL of proteinfactory
10 µL of 500 mM pock (final concentration of 5 mM)
20 µM MaPylRs (final concentration)
20 µM tRNA ^{pyl}_{CUA} (final concentration)
Target gene template PCR product 30 µL
28 °C, overnight reaction
The target protein was obtained, see FIG. 11.

### Example 8: Construction of Triple-Site Incorporation of Reporter Gene

The codons of the three sites of GFP were mutated.

With the reporter gene inserted at two sites as a template, Thr at position 105 of EGFP was mutated into stop codon TAG.

### Primer:

Primer 15105TAGF:
   CAAGGACGACGGTTAGTACAAGACCAGAGCTGAAGTTAAGTTCG (SEQ ID No: 20)
Primer 16105TAGR: (SEQ ID No: 21)
   CAGCTCTGGTCTTGTACTAACCGTCGTCCTTGAAAGAGATG

The PCR reaction and the transformation operation of the ligation product were the same as those in Example 3, and a triple-site mutation template sequence (SEQ ID No: 1) was obtained.

### Example 9: Synthesis of Target Protein with Triple-Site Incorporation of Reporter Gene

6.1 Insertion of non-natural amino acids at three sites of GFP protein in a cell-free system was achieved.

*In vitro* translation conditions:
ProteinFactory was dissolved by ddH2O.
1 mL of proteinfactory
10 µL of 500 mM pock (final concentration of 5 mM)
20 µM MaPylRs (final concentration)
20 µM tRNA ^{pyl}_{CUA} (final concentration)
Target gene template PCR product 30 µL

The mixture was reacted overnight at 28 °C to synthesize a target protein with triple-site incorporation of the reporter gene (see FIG. 13).

Example 10: Further Verification Experiments for Insertion of Non-Natural Amino Acid into Proteins.
1. The expression product after the introduction of the non-natural amino acids (Examples 7 and 9) was centrifuged at 15000 rpm for 15 min, and the supernatant was collected, added with 100 µL of HisMonsterBeads (Kangma PROTN_HMBN1V00001), and incubated at 4 °C for 30 min. The magnetic beads were adsorbed on a magnetic rack and washed 3 times with a wash buffer of 50 mM Tris-HCI pH 8.0, 500 mM NaCl and 20 mM imidazole, and finally the sample was eluted with 50 µL of elution buffer: 50 mM Tris-HCI pH 8.0, 500 mM NaCl and 250 mM imidazole.

The sample was divided into two equal aliquots, one of which was added with 1/10 volume of DNA loading buffer. 1/4 volume of 5× SDS-PAGE loading buffer was added to the other one, and the mixture was incubated at 95 °C for 5 min. The above were the denatured sample and non-denatured sample, respectively. Electrophoretic separation was performed using an 8-16 % gradient precast gel (Wansheng Haotian, GSH2001-816T). The non-denatured sample was placed on a gel imager after electrophoresis, and the expression of EGFP protein was confirmed in the cy3 channel under the fluorescence photographing mode.

The denatured sample was subjected to electrophoresis, stained with Coomassie brilliant blue, and decolored. The band with the correct molecular weight was cut off and subjected to LC-MS/MS mass spectrometry assay, and the insertion of non-natural amino acids could be confirmed (FIGs. 12 and 13).

2. The effect of the triple-site insertion was further verified. As shown in FIG. 6, it represents the fluorescence image of the protein synthesized by the ncaa triple-site insertion gene. 1 is the expression supernatant (MW = 35 KDa) of the GFP gene with triple-site TAG(3 sites) in a cell-free *in vitro* orthogonal translation system. 2 is the expression supernatant (MW = 58.5 KDa) fused with a TEV enzyme-TEV enzyme cutting site (with a TAG mutation)-EGFP gene. The construct had an insertion site for a non-natural amino acid, and GFP can be expressed only when the non-natural amino group is inserted into a synthetic polypeptide chain, which had green fluorescence. Also, the TEV enzyme cutting site had non-natural amino acid mutation and therefore cannot be recognized by TEV. The molecular weight of the fusion protein was 58.5 KDa. 3 is a sample of 1 purified by a Ni column. 4 is a sample of 2 purified by a Ni column. From this, it can be seen that the results show that the GFP gene with 3 TAG stop codons can also be expressed in a cell-free *in vitro* orthogonal translation system. The fusion protein with a TEV label also indicates that TAG can introduce non-natural amino acids into the polypeptide chain of a protein.

## Claims

1. An *in-vitrocell-free* protein synthesis system for inserting a non-natural amino acid, wherein the reaction system comprises:
(1) a cell extract;
(2) a non-natural amino acid;
(3) an exogenous orthogonal aminoacyl tRNA synthetase/orthogonal tRNA pair; and (4) a template comprising a target protein gene sequence, wherein at least one codon encoding an amino acid in the target protein gene sequence is mutated into a stop codon.

2. The *in-vitro* cell-free protein synthesis system for inserting a non-natural amino acid according to claim 1, wherein the stop codon is TAG.

3. The *in-vitro* cell-free protein synthesis system for inserting a non-natural amino acid according to claim 1 or 2, wherein the cell extract is preferably selected from any one of the following sources: *Escherichia coli,* a yeast cell, a mammalian cell, a plant cell, an insect cell, and combinations thereof; the yeast cell is preferably selected from one of *Pichia pastoris, Pichia finlandica, Pichia trehalophila, Pichia koclamae, Pichia membranaefaciens, Pichia minuta* (methanol-inducible yeast (*Ogataea minuta*) and *Pichia lindneri*)*, Pichia opuntiae, Pichia thermotolerans, Pichia salictaria, Pichia g uercuum, Pichia pijperi, Pichia stiptis, Pichia methanolica, Pichia sp., Saccharomyces cerevisiae, Saccharomyces sp., Hansenula polymorpha, Kluyveromyces, K. lactis, Kluyveromyces marxianus, Kluyveromyces marxianus var.lactis, Kluyveromyces marxianus var.marxianus, Kluyveromyces marxianus var.vanudenii, Kluyveromyces dobzhanskii, Kluyveromyces aestuarii, Kluyveromyces nonfermentans, Kluyveromyces wickerhamii, Kluyveromyces thermotolerans, Kluyveromyces fragilis, Kluyveromyces hubeiensis, Kluyveromyces polysporus, Kluyveromyces siamensis,* and *Kluyveromyces yarrowii,* and combinations thereof.

4. The *in-vitro* cell-free protein synthesis system for inserting a non-natural amino acid according to any one of claims 1-3, wherein a structural formula of the non-natural amino acid is a compound of formula (I), 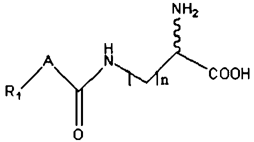 wherein n is selected from natural numbers of 1-20, R₁ is selected from substituted or unsubstituted C5-C60 aryl or heteroaryl, substituted or unsubstituted C1-C20 alkyl, substituted or unsubstituted C2-C20 alkenyl, and substituted or unsubstituted C2-C20 alkynyl, and A is selected from O and -CH₂-.

5. The *in-vitro* cell-free protein synthesis system for inserting a non-natural amino acid according to claim 4, wherein n is selected from natural numbers of 1-10, and R 1 is selected from substituted or unsubstituted C2-C20 alkenyl and substituted or unsubstituted C2-C20 alkynyl; preferably, n is selected from natural numbers of 1-6, and R₁ is selected from substituted or unsubstituted C2-C10 alkenyl and substituted or unsubstituted C2-C10 alkynyl; further preferably, R₁ is selected from substituted or unsubstituted C2-C6 alkynyl.

6. The *in-vitro* cell-free protein synthesis system for inserting a non-natural amino acid according to claim 4 or 5, wherein the structural formula of the non-natural amino acid is selected from one and combinations of the following: 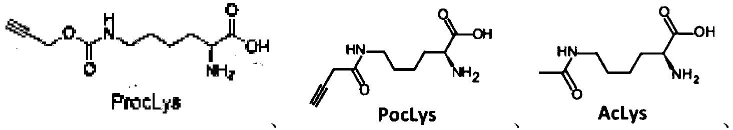 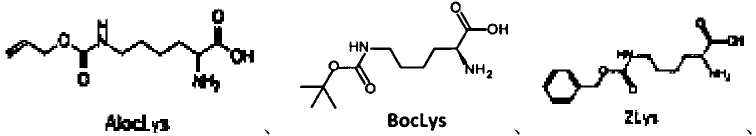 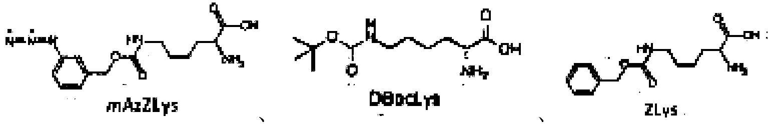 and

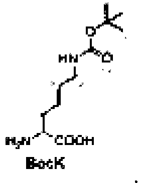

7. The *in-vitro* cell-free protein synthesis system for inserting a non-natural amino acid according to any one of claims 1-6, wherein the exogenous orthogonal aminoacyl tRNA synthetase is selected from natural or mutated Pyl-tRNA synthetase (PylRS), Leu-tRNA synthetase (LeuRS), Tyr-tRNA synthetase (TyrRS), Phe-tRNA synthetase (PheRS), and TrP-tRNA synthetase (TrpRS); the tRNA is selected from natural or mutated tRNA_{Pyl}, tRNA_{Leu}, tRNA_{Tyr}, tRNA_{Phe}, and tRNA_{Trp}.

8. The *in-vitro* cell-free protein synthesis system for inserting a non-natural amino acid according to any one of claims 1-7, wherein the exogenous orthogonal aminoacyl tRNA synthetase is selected from natural or mutated MaPylRS, MmPylRS, MbPylRS, EcTyrRS, MjTyrRS, EcLeuRS, ScPheRS, ScTrpRS, and BsTrpRS, preferably MaPylRS.

9. The *in-vitro* cell-free protein synthesis system for inserting a non-natural amino acid according to any one of claims 1-8, wherein the target protein is selected from fluorescein protein, luciferase, green fluorescent protein, yellow fluorescent protein, red fluorescent protein, glyceraldehyde-3-phosphate dehydrogenase, catalase, actin, a variable region of an antibody, a luciferase mutation, α-amylase, enterocin A, hepatitis C virus E2 glycoprotein, insulin precursor, interferon αA, interleukin-1β, lysozyme, serum albumin, single-chain variable fragment (scFV), transthyretin, tyrosinase, xylanase, and combinations thereof.

10. The *in-vitro* cell-free protein synthesis system for inserting a non-natural amino acid according to any one of claims 1-9, further comprising: one or more of a buffer, potassium ion, magnesium ion, polyethylene glycol, an optional aqueous solvent, and a phosphate.

11. The *in-vitro* cell-free protein synthesis system for inserting a non-natural amino acid according to claim 10, wherein the system further has one or more of the following characteristics:
(1) the relative v/v of the cell extract to the reaction system is 20%-80%;
(2) polyethylene glycol is further included, and the relative w/v of the polyethylene glycol to the reaction system is 0.1%-8%, preferably 0.5%-4%, and more preferably 1%-2%;
(3) the relative concentration of the exogenous orthogonal aminoacyl tRNA synthetase to the reaction system is in a range of 0.001-1 mmol/L, preferably 0.005-0.1 mmol/L, and more preferably 0.005-0.05 mmol/L;
(4) the relative concentration of the orthogonal tRNA to the reaction system is in a range of 0.001-1 mmol/L, preferably 0.005-0.1 mmol/L, and more preferably 0.02-0.1 mmol/L.

12. A kit, comprising the reaction system according to any one of claims 1-11.

13. A method for synthesizing a protein containing a non-natural amino acid using an *in-vitro* cell-free system, wherein the preparation is performed using the synthesis system according to any one of claims 1-11 or the kit according to claim 12.

14. Use of the system according to any one of claims 1-11 or the kit according to claim 12 in the synthesis of a protein containing a non-natural amino acid.

15. A protein containing a non-natural amino acid, prepared by the synthesis system according to any one of claims 1-11 or the kit according to claim 12 or the method according to claim 13.

16. Use of the protein containing a non-natural amino acid according to claim 15 in click chemistry modification.
